(19) Europäisches Patentamt
European Patent Office
Office européen des brevets

(11) Publication number: **0 002 933**
**A1**

# (12) EUROPEAN PATENT APPLICATION

(21) Application number: **78300874.1**

(22) Date of filing: **20.12.78**

(51) Int. Cl.²: **G 01 N 33/16**

(30) Priority: **27.12.77 US 864724**

(43) Date of publication of application: **11.07.79**
**Bulletin 79/14**

(84) Designated Contracting States: **BE CH DE FR GB IT NL SE**

(71) Applicant: **Gosalvez, Mario Gosalvez, Caleruega 21 7a Pinar de Chamartin, Madrid (ES)**

(72) Inventor: **Gosalvez, Mario Gosalvez, Caleruega 21 7a Pinar de Chamartin, Madrid (ES)**

(74) Representative: **Collier, Jeremy Austin Grey et al, J.A.Kemp & Co. 14, South Square Gray's Inn, London WC1R 5EU (GB)**

(54) **Method for assessing cancerous malignancy or benignity and diagnostic pack for use therein.**

(57) The relative malignancy or benignity of a sample of cells suspected of being cancerous is determined by assaying intracellular constituents of the said cells to determine the rate of aerobic glycolysis as a function of the degree to which pyruvate kinase in the cells is inhibited by the presence of adenosine triphosphate. By this method an essentially quantitative assessement of the degree of malignancy or benignity may be made whithout the uncertainties which accompany histological assessement.

EP 0 002 933 A1

0002933



DESCRIPTION

"METHOD FOR ASSESSING CANCEROUS MALIGNANCY OR BENIGNITY AND DIAGNOSTIC PACK FOR USE THEREIN"

This invention relates to the detection of cancerous diseases.

The cancerous diseases, including both leukaemia and malignant neoplasias, rank third in the causes of human death. The progression of neoplasia in its invasiveness of healthy tissue depends mainly on the cell growth rate of the neoplastic cells. Although other factors such as tumour localisation, and the subject's general state of health and immunological resistance are relevant, the malignancy of a tumour or a leukaemia is principally dependent on, and is directly proportional to, the growth rate of its cells. Heretofore, the only generally accepted method of detecting tumour cell malignancy has been through observation of the morphology of the tumour cells. Undifferentiated cell morphology and abnormal nuclear apparatus are common in cells which, like the undifferentiated embryonic cells, exhibit a high growth rate or rate of multiplication. The histological or qualitative morphological method previously used for the diagnosis of tumour malignancy is not completely satisfactory, owing to its reliance on the visual perception by pathologists of ill-defined

morphological signs and the fact that, commonly, surgical, prognostic and other decisions are made on the basis of the inspection of only a few hundred milligrams of suspect material. Histological examination may be supplemented by, e.g., immunological matching between tumour and host, although even this corroborative evidence, whether or not accompanied by attention to clinical signs, can sometimes prove insufficient or even misleading. When it is recognised that many therapeutic, economic and social decisions in the case of cancer patients are based entirely on the diagnosis of a cancer's malignancy and the resulting prognosis, it will be appreciated that a need has arisen for a true, quantitative and precise method for assessing relative degrees of malignity or benignity based on determination of cell growth rate. This need is especially significant in the case of tumours such as intestinal polyps and ovarian and mammary tumours which frequently exhibit great cellular growth rate, yet appear morphologically benign. In such cases, morphological evaluation alone may lead to fatal delays when surgical decisions must be made.

Aerobic glycolysis (the production of lactic acid from glucose in the presence of oxygen) appears to be a unique metabolic characteristic of cancerous and embryonic cells. Biochemists generally agree that high aerobic glycolysis is associated with a high degree of biochemical undifferentiation, and it has been generally accepted that the rate of aerobic glycolysis in tumour cells closely correlates with cell growth rate.

I have discovered that aerobic glycolysis in cancer cells is attributable to the failure of adenosine triphosphate (ATP) to inhibit the glycolytic enzyme pyruvate kinase. In normal cells, in the presence of oxygen, ATP generated by the mitochondria essentially

completely allosterically inhibits two enzymes in the metabolic pathway from glucose to lactic acid, i.e., phosphofructokinase and pyruvate kinase. Accordingly, in normal tissue no glycolysis occurs in the presence of oxygen. However, it now appears that in tumour cells ATP only partially inhibits phosphofructokinase, and that it inhibits pyruvate kinase either partially or not at all. Known methods may be applied to the determination of the extent to which enzymes in this metabolic pathway are inhibited by ATP. I have discovered a high degree of correlation between, e.g., the extent of ATP inhibition of pyruvate kinase in cancer cells and the Crabtree and Pasteur effects exhibited by these cells. Moreover, when the percentage inhibition by ATP of pyruvate kinase in cell samples under assay is compared with the malignancy or benignity of cancers in subjects from which the cells were drawn, as determined by morphological, biological and clinical study of the disease's evolution, a high degree of correlation emerges. Table I below compares the relative malignancy of cancers in subjects of various species with the percent ATP inhibition of pyruvate kinase in cancer cell homogenates derived from those subjects.

TABLE I

| TISSUE | SPECIES | MAXIMUM % INHIBITION OF PYRUVATE KINASE BY ATP | MALIGNANCY |
|---|---|---|---|
| Normal tissue | Rat | 99% | No cell growth |
| Normal liver | Human | 99% | No cell growth |
| Mammary fibro-adenoma | Rat | 49% | Very benign |
| Cerebral ependonoma | Human | 40% | Benign |
| Erhlich ascites tumour (Rome Strain) | Mouse | 39% | Slow growth rate |
| Acute lympho-blastic leukaemia | Human | 32% | Moderately malign |
| Leukaemia P-P388 | Mouse | 25% | Malign |
| Walter Carcino-sarcoma | Rat | 22% | Malign |
| Mammary carcinoma | Human | 17% | Malign |
| Leukaemia 1210 | Mouse | 16% | Very malign |

TABLE I (continued)

| TISSUE | SPECIES | MAXIMUM % INHIBITION OF PYRUVATE KINASE BY ATP | MALIGNANCY |
|---|---|---|---|
| Glioma | Human | 15% | Malign |
| Ovarian carcinoma | Human | 10% | Malign |
| Multiform glioma | Human | 4% | Very malign |
| Erhlich ascites (Paris strain) | Mouse | 4% | Very high growth rate |

Additional experimental evidence for the profound effect of ATP inhibition of pyruvate kinase drawn from cancerous cells is presented in M. Gosalvez, et. al., Cancer Research 38, 142-148, (1978).

The present invention accordingly provides a method for assessing the cancerous malignancy or benignity of cells in a cell sample which comprises determining whether the activity of pyruvate kinase in the said cells is inhibited by the presence of adenosine triphosphate. As usually carried out this method involves (a) preparing from the said sample a homogenate containing pyruvate kinase from the said cells; and (b) determining whether the action of the said pyruvate kinase on its substrate phosphoenol pyruvate is inhibited to any degree by the presence of adenosine triphosphate.

In more detail, the theoretical basis of this new method is as follows. The step-wise degradation of glucose to pyruvic acid, according to the well-known Embden-Myerhof pathway, includes the

reaction:

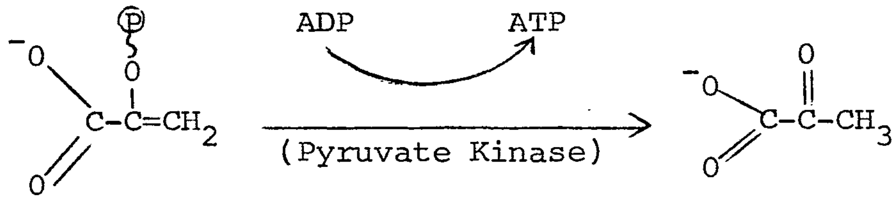


Phosphoenol-pyruvate

Pyruvate

wherein ADP and ATP are, respectively, adenosine diphosphate and adenosine triphosphate. In healthy cells this reaction is suppressed by ATP inhibition of the enzyme pyruvate kinase. I have discovered, as previously noted, that in cancerous cells the reaction proceeds in greater or less degree, correlative to the degree of malignancy, in proportion to the extent to which ATP fails to inhibit pyruvate kinase activity. A preferred assay, therefore, involves assessment of the progress of the foregoing reaction in the presence of various levels of ATP calculated to reveal the extent, if any, by which sample-derived pyruvate kinase activity is inhibited by ATP. The extent of such inhibition can be characterised on a percentage scale relative to the foregoing reaction, taking 0% as no inhibition, 100% as total inhibition, and "maximum percent inhibition of pyruvate kinase activity" as that point on the scale beyond which addition of further ATP essentially fails to affect further the progress of the reaction.

Assay for the activity of sample-derived pyruvate kinase may be commenced at any point prior to the phosphoenolpyruvate conversion on the Embden-Meyerhof pathway (as to which see, e.g., J. D. Watson,

Molecular Biology of the Gene, pp. 34-35, (3rd ed. 1977) W.A. Benjamin, Inc., publ., Menlo Park, Ca.), provided any necessary substrates, co-factors, enzymes and co-enzymes are supplied to the assay medium. (As used herein, the term "pyruvate kinase assay medium" means a solution containing the active ingredients necessary for activity assay, other than subsequently added ATP). Preferably, however, the phosphoenolpyruvate reaction is directly monitored by supplying phosphoenolpyruvate and ADP to the sample-derived pyruvate kinase, and following the resulting reaction, preferably spectrophotometrically, in the presence of varying amounts of ATP.

It is especially convenient therefore to supply the user of the new method with a diagnostic pack in accordance with the invention which comprises (a) a medium for pyruvate kinase activity assay; and (b) adenosine triphosphate.

An especially preferred method of following the reaction spectrophotometrically involves compound optical assay, in which the influence of the reaction under study on a secondary reaction with an observable end product is followed. See Felu, FEBS Letters 50, 334 (1975) and Bucher, T. et al., in : Methods in Enzymology, Vol. I (S.P. Colowick and N.O. Kaplan, eds.) Academic Press, N.Y. p. 435. Thus, in the compound reaction:

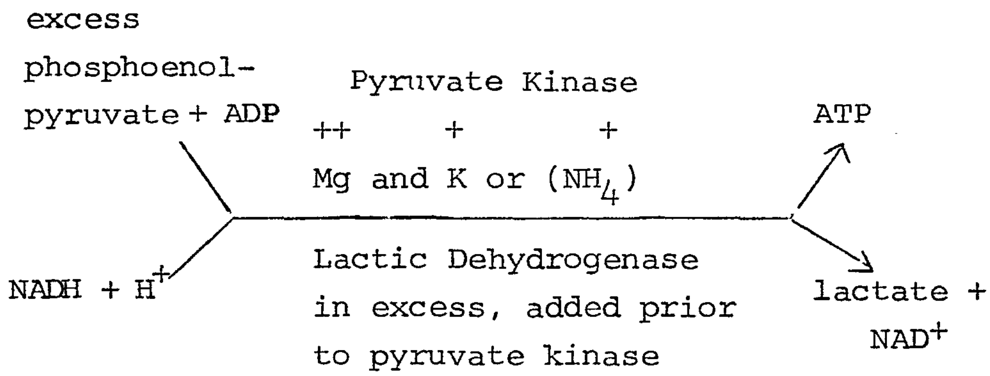


pyruvate kinase activity can be monitored by observing the rate of disappearance of the band (at 340 m μ) of the hydrogen donor NADH (nicotinamide adenine dinucleotide, reduced form). Successive repetitions of the assay with differing amounts of added ATP establish the point beyond which ATP addition fails to influence the rate of disappearance of the hydrogen donor. Thus, for example, a graphical comparison of the decrease in absorbance in the absence of ATP and at various concentrations of ATP, shows the maximum point of allosteric inhibition at the point of inflexion of the graph.

Samples for assay may be obtained as whole tissue (e.g., of volume 1-10 $mm^3$) in the case of suspected tumours, by intraoperatorybiopsy or needle biopsy. In the case of suspected blood malignancies, the collection of white blood cells by differential centrifugation is sufficient. Samples are preserved on ice pending the preparation of homogenates suitable for assay. Homogenisation frees cell-contained pyruvate kinase for assay and involves cell disruption by conventional techniques which avoid, to a useful degree, the destruction of nuclei, mitochondria and other cell particulates. Typically, the sample may be placed in a hand homogeniser which is supplied with

the assay kit, in buffered isotonic solution, and homogenised with several strokes of a pestle. From, e.g., 0.1 to 1 ml of buffered solution, depending on the size of the sample, is preferably employed. As one of many appropriate buffers may be mentioned a solution comprising 50 mM Tris-HCl, pH 7.0, 0.1 mM EDTA and 0.5 mM dithioerythritol. In the preferred embodiment of the invention the homogenate is next centrifuged, for example at about 5,000-10,000 rpm in a conical centrifuge tube for 10 minutes, and the supernatant is dialysed against water (e.g., for 2 hours at about 2-4°C.). The contents of the dialysis bag are then transferred to a tube immersed in ice. In the case of large samples, the centrifugation and dialysis steps may be omitted, as will be appreciated by the art-skilled. Preferably, however, a dialysis bag is supplied with the kit. As used herein, the term "homogenate" refers to a free enzyme-containing product of homogenisation, whether or not accompanied by other cellular debris.

In the most preferred embodiment of the invention, the kit or diagnostic pack includes pyruvate kinase assay medium which, when diluted 5 to 10 times with distilled water, has the composition 50 mM Imidazole HCl, pH 7.0, 0.1 M KCl, 5 mM $MgCl_2$, 0.15 mM NADH, 1 mM Mg ADP, 2 mM phosphoenolpyruvate and 1 unit per ml of lactic dehydrogenase. A small sample of homogenate is added to, e.g., 3 ml of assay medium in a cuvette, to which ATP is also added from a third stock solution for NADH absorbance determinations at 340 mu. In successive determinations, the different ATP concentrations initially in the assay medium may be, e.g., 0.2, 0.5, 1, 2, 3, 4 and 5 mM. A plot of decrease in absorbance against ATP concentration, shows the maximum percentage inhibition of pyruvate kinase

by ATP. Alternatively, as those skilled in the art will appreciate from the foregoing, a "yes-no" result may be determined by comparing pyruvate kinase inhibition at a particular, optimal ATP concentration to values previously determined at that concentration with samples, of known malignancy, of each different type of cancer under assay.

In its broadest aspect, my invention is based on the recognition that the susceptibility of cell-derived pyruvate kinase to ATP inhibition is correlated to the relative cell growth rate and degree of undifferentiation, and hence malignancy, of cells from which the enzyme is derived. The invention accordingly affords a quantitative biochemical method for the diagnosis and prognosis of cancerous diseases.

CLAIMS

1. A method for assessing the cancerous malignancy or benignity of cells in a cell sample which comprises determining whether the activity of pyruvate kinase in the said cells is inhibited by the presence of adenosine triphosphate.

2. A method according to claim 1 for assessing the cancerous malignancy or benignity of cells in a cell sample which comprises:

(a) preparing from the said sample a homogenate containing pyruvate kinase from the said cells; and

(b) determining whether the action of the said pyruvate kinase on its substrate phosphoenol pyruvate is inhibited to any degree by the presence of adenosine triphosphate.

3. A method according to claim 1 or 2 wherein the maximum extent to which pyruvate kinase is inhibited by adenosine triphosphate is determined.

4. A method according to any of claims 1 to 3 wherein the said determination is carried out spectrophotometrically.

5. A method according to claim 4 wherein the said determination is carried out by compound optical assay.

6. A method according to any of claims 1 to 5 wherein the sample and adenosine triphosphate are added to a pyruvate kinase assay medium comprising phosphoenol pyruvate, adenosine diphosphate, lactic dehydrogenase and a hydrogen donor, and the determination is carried out by monitoring the rate of hydrogen donor disappearance spectrophotometrically.

7. A method according to claim 6 wherein the hydrogen donor is the reduced form of nicotinamide adenise dinucleotide.

8. A method according to claim 6 or 7 wherein the determination is repeated using different amounts of added adenosine triphosphate so as to determine the point beyond which addition of further adenosine triphosphate essentially ceases to affect the rate of disappearance of the hydrogen donor.

9. A method according to claim 8 wherein the rate of disappearance of hydrogen donor in the absence of any added adenosine triphosphate is also determined.

10. A diagnostic pack for use in the assessment of the cancerous malignancy or benignity of cells in a cell sample which comprises:

(a) a medium for pyruvate kinase activity assay; and

(b) adenosine triphosphate.

11. A diagnostic pack according to claim 10 wherein the said medium is a medium for compound optical assay for pyruvate kinase activity.

12. A diagnostic pack according to claim 11 wherein the said medium comprises lactic dehydrogenase, phosphoenolpyruvate, adenosine diphosphate, and a hydrogen donor.

13. A diagnostic pack according to claim 12 wherein the said hydrogen donor is the reduced form of nicotinamide adenise dinucleotide.

14. A diagnostic pack according to any of claims 10 to 13 which additionally comprises a quantity of buffered isotonic solution for the preparation of a cell homogenate.

3

European Patent Office

# EUROPEAN SEARCH REPORT

0002933

Application number

EP 78 300 874.1

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | G 01 N 33/16 |
| A | DE - B - 2 412 354 (BOEHRINGER MANNHEIM)<br>-- | | |
| A | US - A - 3 575 811 (E.W. CAHPPELLE et al)<br>-- | | TECHNICAL FIELDS SEARCHED (Int.Cl.²) |
| A | S.M. RAPOPORT "Medizinische Biochemie"<br>1962<br>VEB Verlag Volk und Gesundheit, Berlin,<br>page 405 to 409<br>---- | | G 01 N 33/16 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 20-03-1979 | SCHWARTZ |